# EUROPEAN PATENT SPECIFICATION

(11) **EP 0 465 024 B1**
(45) Date of publication and mention of the grant of the patent: **05.02.1997**
(21) Application number: 91305310.4
(22) Date of filing: 12.06.1991
(51) Int. Cl.: C12N 15/82, C12N 15/29, A01H 1/02

(54) **Control of microsporogenesis using externally inducible promoter sequences**
Kontrolle der Mikrosporogenese durch Verwendung von externinduzierbaren Promotorsequenzen
Contrôle de microsporogénèse utilisant des séquences de promoteurs inductibles de l'extérieur

(30) Priority: 12.06.1990 US 537183
(43) Date of publication of application: 08.01.1992
(73) Proprietor: PIONEER HI-BRED INTERNATIONAL, INC., Des Moines Iowa 50309 (US)
(72) Inventor: Albertsen, Marc C., Ankeny, IA 50021 (US); Beach, Larry R., Des Moines, IA 50311 (US); Howard, John, West Des Moines, IA (US); Huffman, Gary A., Des Moines, IA 50311 (US)
(74) Representative: Goldin, Douglas Michael

(56) References cited:
- EP-A- 0 329 308
- WO-A-89/10396

## Description

### Technical Field

The present invention relates to the use of microsporogenesis genes and inducible promoters for the production of hybrid seed.

### Background Art

The goal of plant breeding is to combine in a single variety/hybrid various desirable traits of the parental lines. For field crops, these traits may include resistance to diseases and insects, tolerance to heat and drought, reducing the time to crop maturity, greater yield, and better agronomic quality. With mechanical harvesting of many crops, uniformity of plant characteristics such as germination and stand establishment, growth rate, maturity, and fruit size, is important.

Field crops are bred through techniques that take advantage of the plant's method of pollination. A plant is self-pollinating if pollen from one flower is transferred to the same or another flower of the same plant. A plant is cross-pollinated if the pollen comes from a flower on a different plant.

Plants that have been self-pollinated and selected for type for many generations become homozygous at almost all gene loci and produce a uniform population of true breeding progeny. A cross between two homozygous lines produces a uniform population of hybrid plants that may be heterozygous for many gene loci. A cross of two plants each heterozygous at a number of gene loci will produce a population of hybrid plants that differ genetically and will not be uniform.

Maize plants (Zea mays L.) can be bred by both self-pollination and cross-pollination techniques. Maize has male flowers, located on the tassel, and female flowers, located on the ear, on the same plant. Natural pollination occurs in maize when wind blows pollen from the tassels to the silks that protrude from the tops of the incipient ears.

The development of maize hybrids requires the development of homozygous inbred lines, the crossing of these lines, and the evaluation of the crosses. Pedigree breeding and recurrent selection are two of the breeding methods used to develop inbred lines from populations. Breeding programs combine desirable traits from two or more inbred lines or various broad-based sources into breeding pools from which new inbred lines are developed by selfing and selection of desired phenotypes. The new inbreds are crossed with other inbred lines and the hybrids from these crosses are evaluated to determine which have commercial potential.

Pedigree breeding starts with the crossing of two genotypes, each of which may leave one or more desirable characteristics that is lacking in the other or which complement the other. If the two original parents do not provide all of the desired characteristics, other sources can be included in the breeding population. In the pedigree method, superior plants are selfed and selected in successive generations. In the succeeding generations the heterozygous condition gives way to homogeneous lines as a result of self-pollination and selection. Typically in the pedigree method of breeding five or more generations of selfing and selection is practiced. F₁-->F₂; F₂-->F₃; F₃-->F₄; F₄-->F₅, etc.

A hybrid maize variety is the cross of two inbred lines, each of which may have one or more desirable characteristics lacked by the other or which complement the other. The hybrid progeny of the first generation is designated F₁. In the development of hybrids only the F₁ hybrid plants are sought. The F₁ hybrid is more vigorous than its inbred parents. This hybrid vigor, or heterosis, can be manifested in many ways, including increased vegetative growth and increased yield.

The development of a hybrid maize variety involves three steps: (1) the selection of superior plants from various germplasm pools; (2) the selfing of the superior plants for several generations to produce a series of inbred lines, which although different from each other, each breed true and are highly uniform; and (3) crossing the selected inbred lines with unrelated inbred lines to produce the hybrid progeny (F₁). During the inbreeding process the vigor of the lines decreases. Vigor is restored when two unrelated inbred lines are crossed to produce the hybrid progeny (F₁). An important consequence of the homozygosity and homogeniety of the inbred lines is that the hybrid between any two inbreds will always be the same. Once the inbreds that give the best hybrid have been identified, the hybrid seed,can be reproduced indefinitely as long as the homogeneity of the inbred parents is maintained.

A single cross hybrid is produced when two inbred lines are crossed to produce the F₁ progeny. A double cross hybrid, is produced from four inbred lines crossed in pairs (A x B and C x D) and then the two F₁ hybrids are crossed again (A x B) x (C x D). Much of the hybrid vigor exhibited by F₁ hybrids is lost in the next generation (F₂). Consequently, seed from hybrid varieties is not used for planting stock. Likewise, it is very important in the production of hybrid seed to avoid self-pollination and the production and sale of inbred seed to end users.

Hybrid maize seed can be produced by manual detasseling. Alternate strips of two inbred varieties of maize are planted in a field, and the pollen-bearing tassels are removed from one of the inbreds (female). Providing that there is sufficient isolation from sources of foreign maize pollen, the ears of the detasseled inbred will be fertilized only with pollen from the other inbred (male), and the resulting seed is therefore hybrid and will form hybrid plants. Unfortunately, the manual detasseling process is not entirely reliable. Occasionally a female plant will be blown over by a storm and escape detasseling. Or, a detasseler will not completely remove the tassel of the plant. In either event, the female plant will successfully shed pollen and some female plants will be self-pollinated. This will result in seed of the female inbred being harvested along with the hybrid seed which is normally produced.

Alternatively, the female inbred can be mechanically detasseled. Mechanical detasseling is approximately as reliable as manual detasseling, but is faster and less costly. However, most detasseling machines produce more damage to the plants than manual detasseling. Thus, no form of detasseling is presently entirely satisfactory, and a need continues to exist for alternatives which further reduce production costs and the eliminate self-pollination in the production of hybrid seed.

The laborious detasseling process can be avoided by using cytoplasmic male-sterile (CMS) inbreds. Plants of a CMS inbred are male sterile as a result of cytoplasmic factors resulting from the cytoplasmic, as opposed to the nuclear, genome. Thus, this characteristic is inherited exclusively through the female parent, since only the female provides cytoplasm to the fertilized seed. CMS plants are fertilized with pollen from another inbred that is not male-sterile. Pollen from the second inbred may or may not contribute genes that make the hybrid plants male-fertile. Usually seed from detasseled normal maize and CMS produced seed of the same hybrid must be blended to insure that adequate pollen loads are available for fertilization when the hybrid plants are grown.

There can be other drawbacks to CMS. One is an historically observed association of a specific variant of CMS with susceptibility to certain crop diseases. This problem has led to virtual abandonment of use of that CMS variant in producing hybrid maize. In addition, CMS sometimes has a negative association with agronomic performance, particularly in the areas of stalk quality, early seedling vigor, and yield. Finally, CMS exhibits on occasion the potential for breakdown of sterility in certain environments, rendering CMS lines unreliable for hybrid seed production.

Another form of sterility, genic male sterility, is disclosed in U.S. Patents 4,654,465 and 4,727,219 to Brar et al. However, this form of genetic male sterility requires maintenance of multiple mutant genes at separate locations within the genome and requires a complex marker system to track the genes and make use of the system convenient.

In self-pollinated species, such as soybeans and cotton, the male and female organs are anatomically juxtaposed. During natural pollination, pollen from the male reproductive organs of a given flower pollinate the female reproductive organs of the same flower. This is in contrast to cross-pollinated species, such as maize, where pollen from the tassel of one plant typically pollinates the silks of another plant through wind dispersal. This can readily occur because of the separation of the male and female reproductive organs. Hybrid production among self-pollinated crops can be difficult because of the close association of the male and female reproductive organs. In addition to the physical difficulty in effecting hybrid production in a self-pollinating crop, the amount of heterosis exhibited in a hybrid is often too low to justify the additional expense required to produce hybrid seed. A reliable form of male sterility would offer the opportunity for improved hybrid plant breeding and increased yields in these species.

### Disclosure of the Invention

The present invention differs from conventional approaches to male sterility in plant breeding and seed production in that an inducible promoter is used to regulate expression of a gene which is known to be critical in microsporogenesis, i.e., the production of pollen. The first step in the practice of this invention is therefore the selection of a gene on which microsporogenesis is dependent.

The selected gene is cloned, its native promoter removed, and the modified gene is inserted into an expression sequence with an inducible promoter responsive to external control. Preferably, the promoter is one which responds to application of a specific non-phytotoxic chemical to the plant.

Using transformation and gene substitution, the "critical" gene is deleted from the genome of the plant and replaced by the genetically-engineered gene incorporated into the expression sequence with the inducible promoter.

This invention is unique in that the inducible promoter is used to induce fertility, not sterility. In this invention, the selected gene's promoter sequences are removed so that the gene is not transcribed and the plant is male sterile. When it is desired to increase the male-sterile plant, male fertility is restored by inducing expression of the critical gene. In the preferred embodiment this is accomplished by treating growing male sterile plants with a specific non-phytotoxic chemical.

It will be appreciated that male sterility could be imparted in a manner by which the "critical gene" is "off" and requires the chemical for expression, or in a manner by which the critical gene is "on" and chemical treatment is necessary to impart sterility. The latter method is described in PCT Publication WO89/10396 of Mariani et al (based on Intl. Appl. No. PCT/EP89/00495). EP-A-0329308 also describes a method for inducing male sterility in a plant by chemical treatment to activate an inducible promoter (an inducible promoter of an anti-sense gene corresponding to a wild-type gene critical to pollen formation or function in the same plant).

Induction of the inducible promoter by chemical treatment will be dependent on various factors associated with the chemical treatment itself and various environmental conditions at the time of treatment. If the critical gene were normally "on," to be inactivated by chemical treatment, a treatment failure would result in self-pollination and production and sale of inbred, rather than hybrid seed. Seed laws that govern the sale of hybrid seed require a high degree of seed purity such that percentages of seed that do not conform to the hybrid specification must be kept very low. Because one maize plant can produce in excess of six million pollen granules, even a limited treatment failure could result in a high percentage of self-pollination. For these reasons, the present invention is practiced in such a manner that the gene is normally "off" and the corresponding trait is not expressed, so that under normal conditions self-pollination cannot occur. In addition, by having the critical gene normally "off," chemical treatment is not necessary in the large-scale production of hybrid seed, so that chemical usage (and associated expense) is minimized and the risk of treatment failure is present only in the carefully controlled, limited scale production of parent seed, where self-pollination is desired. Since treatment failure in such a case results in underproduction of pollen, and since pollen is normally overproduced by a wide margin, the process of this invention for production of parent seed will tolerate a treatment failure rate as high as 70% to 80% with minimal effects on yield of parent seed.

### Industrial Applicability

The procedures for identifying and cloning a male sterile gene are the same as those known in the art to be utilized to clone other genes. The preferred method is transposon (transposable element) tagging because most instances of genetic male sterility in maize are the result of recessive gene mutations. Cloning techniques that require knowledge of the protein sequences of a male sterile gene translation product cannot be used at present because the, gene product of male sterile genes is not yet known.

The procedure for tagging maize genes with transposable elements is known, as reviewed by H. P. Doring, "Tagging Genes with Maize Transposable Elements. An Overview". Maydica 34 (1989): 73-88 and described in U.S. Patent 4,732,856 to Federoff ("Transposable Elements and Process for Using Same"), the disclosures of which are hereby incorporated herein in their entirety. One of the methods by which this is carried out is by intercrossing a maize strain carrying active transposable elements and a dominant allele of the target gene involved in microsporogenesis with a normal maize strain that does not carry transposable elements. Specific gene tagging efficiency can be and preferably is enhanced by positioning the transposable element in the proximity of the target gene locus. Progeny from the intercrosses are selfed and subsequently screened for the most useful mutations. The preferred phenotypes are plants which do not extrude anthers and those which do not produce pollen. Most preferred are phenotypes which do not extrude anthers because this phenotype can easily be screened visually prior to pollination time by gross observation. These male sterile plants represent putative instances in which a transposable element has excised from its original location and has transposed to a locus bearing a gene which is essential for pollen development. Once the transposable element has transposed to such a locus, the gene is inactivated. It will then behave as a recessive gene and result in male sterility. These mutant plants can be crossed to tester stocks for the transposable element to confirm that the element is still present.

Once it has been confirmed that the desired transposable element has transposed into the target gene, genomic clones which hybridize to the transposable element are constructed. The element adjacent sequences of the clones are then used as probes in Southern hybridizations with genomic DNA from strains carrying the mutant allele, the revertant allele, and the wild-type allele. rDNA which reveals the expected differences in size (reflecting the presence or absence of the transposable element) carries the desired modified target gene.

In practice, the frequency with which a particular locus can be targeted with a transposable element usually varies from 10⁻⁵ to 10⁻⁶ (Doring, 1989). However, 100,000 maize plants can easily be grown on an area of less than 10 acres. In addition, under certain circumstances the frequency of the element-induced mutations can be increased. For example, the particular transposable element to be used for gene tagging can be linked to the gene to be tagged by the element. For example, for two different transposable element systems, Ac and Spm/En, the transpositions of these elements occurs preferentially to sites on the chromosome where the element was located before the transposition. Alternatively, different transposable elements have different frequencies of mutation induction. For example, the transposable element called Mutator (Mu) is able to induce new mutations at a frequency 30 to 50 times higher than the frequency in control plants. Additionally, the rate of mutation induction can be influenced by the sex of the element carrying parent. While it cannot be predicted which of the reciprocal crosses will give the higher mutation rate, transposon tagging can readily be performed.

At least seven different maize transposable elements have been cloned at this time. These are Ac, Spm/En, Mu, Tz86, Bs1, rDt, and Mpil. Any of these can be used to clone genes in which a transposable element resides.

Several methods are known in the art for transferring cloned DNA into maize. These include electroporation-facilitated DNA uptake by maize protoplasts (Rhodes et al., "Genetically Transformed Maize Plants from Protoplasts". Science, Vol. 240 (8 April 1988), treatment of maize protoplasts with polyethylene glycol (Lyznik et al., "Stable Co-Transformation of Maize Protoplasts with Gus A and Neo Genes". Plant Molecular Biology 13: 151-161, 1989), and bombardment of maize cells with DNA laden microprojectiles (Klein, et al., Genetic Transformation of Maize Cells by Particle Bombardment". Plant Physiol. (1989) 91, 440-444) and (Klein, et al., "Factors Influencing Gene Delivery into Zea Mays Cells by High-Velocity Microprojectiles". Bio/Technology Vol. 6, May 1988). Each of these techniques has advantages and disadvantages. In each of the techniques, DNA from a plasmid is genetically engineered such that it contains not only the gene of interest, but also selectable and screenable marker genes. A selectable marker gene is used to select only those cells that have integrated copies of the plasmid (the construction is such that the gene of interest and the selectable and screenable genes are transferred as a unit). The screenable gene provides another check for the successful culturing of only those cells carrying the genes of interest. A commonly used selectable marker gene is neomycin phosphotransferase II (NPT II). This gene conveys resistance to kanamycin, a compound that can be added directly to the growth media on which the cells grow. Plant cells are normally susceptible to kanamycin and, as a result, die. The presence of the NPT II gene overcomes the effects of the kanamycin and each cell with this gene remains viable. Another selectable marker gene which can be employed in the practice of this invention is the gene which confers resistance to the herbicide glufosinate (Basta). A screenable gene commonly used is the β-glucuronidase gene (GUS). The presence of this gene is characterized using a histochemical reaction in which a sample of putatively transformed cells is treated with a GUS assay solution. After an appropriate incubation, the cells containing the GUS gene turn blue. Another screenable gene is a transcriptional activator for anthocyanin biosynthesis, as described in the copending application of Bowen et al., U.S.S.N. 387,739, filed August 1, 1989. This gene causes the synthesis of the pigment anthocyanin. Cells transformed with a plasmid containing this gene turn red. Preferably, the plasmid will contain both selectable and screenable marker genes.

The plasmid containing one or more of these genes is introduced into either maize protoplasts or callus cells by any of the previously mentioned techniques. If the marker gene is a selectable gene, only those cells that have incorporated the DNA package survive under selection with the appropriate phytotoxic agent. Once the appropriate cells are identified and propagated, plants are regenerated. Progeny from the transformed plants must be tested to insure that the DNA package has been successfully integrated into the plant genome.

One collection of such mutant genes is already known, and has been described by Albertsen, et al. "Developmental Cytology of 13 Genetic Male Sterile Loci in Maize". Can. J. Genet. Cytol. 23: 195-208, 1981. These are known as male-sterile (ms) genes. These genes affect development of the pollen only; they have no effect on female organ development. These genes disrupt microsporogenesis at characteristic stages of pollen development, rendering the plant male sterile.

Once the mutant gene from any of the foregoing sources has been cloned, it is used as a probe to clone the wild type allele. This is possible because the mutated gene is very closely similar to the wild type allele, and as such, hybridizes to the wild type allele. Once the normal gene has been identified and cloned, the region of the gene known as a promoter region is identified. This region is involved in the start of transcription of that gene.

Genes which are essential to pollen development can also be identified without intermediate use of mutations by isolating mRNA's that are uniquely present during pollen development and constructing a cDNA that can be used to probe a genomic library for the corresponding gene.

In the practice of this invention the promoter region is removed from a cloned gene responsible for male fertility and is replaced with a promoter that only responds to a specific external stimulus. Thus, the gene will not be transcribed except in response to the external stimulus. As long as the gene is not being transcribed, its gene product -- which is necessary for completion of pollen development -- is not produced. This causes a breakdown in one or more of the biochemical/physiologic pathways of pollen development, which results in male sterility. The plant can only become fertile under the specific stimulus that activates the selected promoter.

An example of a responsive promoter system that can be used in the practice of this invention is the glutathione-S-transferase (GST) system in maize. GSTs are a family of enzymes that can detoxify a number of hydrophobic electrophilic compounds that often are used as pre-emergent herbicides (Wiegand, et al., "Messenger RNA Encoding a Glutathione-S-Transferase Responsible for Herbicide Tolerance in Maize is Induced in Response to Safener Treatment". Plant Molecular Biology 7: 235-243, 1986). It has been discovered that treating maize seed with GSTs increases the tolerance of the maize to the herbicides. Studies have shown that the GSTs are directly involved in causing this enhanced tolerance. This action is primarily mediated through a specific 1.1 kb mRNA transcription product. In short, maize has a naturally occurring quiescent gene already present that can respond to GSTs and that can be induced to produce a gene product. This gene has already been identified and cloned. Thus, in one embodiment of this invention, the promoter is removed from the GST responsive gene and attached to the male fertility gene that previously has had its native promoter removed. This engineered gene is the combination of a promoter that responds to an external chemical stimulus and a gene responsible for successful development of fertile pollen.

The gene engineered in the foregoing manner is introduced back into the maize plant through known transformation techniques. The appropriate plant types are selected, that is plants that are male sterile. These plants are male sterile because the isolated and cloned male fertility gene does not have its native promoter and, therefore, is not producing its gene product that is crucial to successful pollen development. Therefore, the engineered gene acts as a recessive mutant allele of that gene. In normal plant biotechnology, once the desired genotype is identified following transformation and regeneration, the plants are selfed to recover that genotype. However, in the practice of this invention, the desired genotype cannot be selfed at the first generation because it is male sterile. To obtain progeny, fertility must be induced by spraying the plants with a compound which induces transcription of the gene by activating the altered promoter. In the case of the GST promoters, the compound is preferably a GST-inducing compound such as N,N-diallyl-2-2-dichloroacetanide. The promoter attached to the male fertility gene responds to this chemical and causes the transcription of the gene to begin. Once this occurs, the normal gene product is produced from the gene and some level of male fertility is induced. Pollen from this plant is then used to effect pollination of the original selected genotype.

Once the initial isolation and propagation of the desired genotype is completed, the procedure is more straightforward. Only inbreds that are used as female parents in hybrid crosses are transformed into male sterile variants. Once they are transformed, the amount of male sterile/female fertile seed must be increased. This is accomplished by planting in an isolated area (away from other maize pollen) and spraying with a chemical to which the promoter responds. Spraying induces the promoter to start transcription of the gene attached to it. This will produce some degree of fertility. A particular advantage of this system in comparison to systems such as that disclosed in PCT Publication WO89/10396 of Mariani et al (based on Intl. Appl. No. PCT/EP89/00495), in which sterility is induced, is that the treatment does not have to be 100% effective, because normally much more pollen is produced by a maize plant than is actually needed for fertilization of all available silks. Therefore, even low fertility restoration will be effective in obtaining acceptable levels of seed increase. At the same time, self-pollination does not occur in hybrid seed production because the plants of this invention are normally male sterile and must be treated to become fertile. In systems in which sterility is induced, induction of sterility must be 100% effective to avoid self-pollination when hybrid seed is produced.

All the seed harvested continues to be homozygous and sterile since the fertility is only restored in a single parent generation by treatment with the fertility inducing chemical. This seed is then used in a hybrid production field where it is used as a female parent. Because the plants are male sterile, they do not have to be detasseled. All of the hybrid plants produced from such seed are male fertile because the resulting progeny inherit one modified gene from the female parent and one normal gene from the male parent. Normal pollen production occurs.

## Claims

1. A method for providing heritable, externally controllable male fertility in a plant, comprising the steps of:
a) selecting a gene'which codes for a gene product on which microsporogenesis in the plant is dependent;
b) cloning the selected gene;
c) linking the cloned gene in an expression sequence with an inducible promoter responsive to external control;
d) causing the gene which codes for the gene product of said cloned gene in the native nuclear genome of the plant to be inoperative; and
e) inserting said expression sequence into the nuclear genome of the plant.

2. A method of reproducing a plant having heritable, externally controllable male fertility resulting from replacement of a gene which codes for a gene product on which microsporogenesis is dependent with a gene which codes for the same gene product, but which is linked in an expression sequence with an inducible promoter responsive to external control, comprising the steps of:
a) planting seed of the plant to provide growing, male-sterile plants;
b) inducing conversion of the growing plants to male fertile form by growing the plants under conditions which induce said promoter, thereby producing the gene product on which microsporogenesis is dependent;
c) open-pollinating the growing plants in isolation to produce seed; and
d) harvesting the seed.

3. A method of producing hybrid seed, comprising the steps of:
a) obtaining seed of a female parent line having male sterility by a method as claimed in claim 2;
b) planting, in cross pollinating juxtaposition, a first seed from a selected male fertile male parent line and a second seed obtained as in (a);
c) growing the seed to mature plants under conditions which maintain male sterility of the plant derived from said second seed;
d) cross pollinating the male sterile female plant with pollen from the male-fertile male plant; and
e) harvesting hybrid seed from the male-sterile female plant.

## Patentansprüche

1. Verfahren zum Erhalt einer vererbbaren, extern kontrollierbaren männlichen Fruchtbarkeit in einer Pflanze, mit den folgenden Stufen:
a) Auswahl eines Genes, welches für ein Genprodukt codiert, von welchem die Mikrosporogenese der Pflanze abhängig ist;
b) Klonierung des ausgewählten Genes;
c) Verbindung des clonierten Genes in einer Expressionssequenz mit einem induzierbaren Promotor, welcher auf externe Kontrolle reagiert;
d) Bewirken daß das Gen, welches für das Genprodukt des genannten clonierten Genes codiert, in dem natürlichen Kerngenom der Pflanze inoperativ ist; und
e) Insertion der genannten Expressionssequenz in das Kerngenom der Pflanze.

2. Verfahren zur Vermehrung einer Pflanze, welche vererbbare, extern kontrollierbare männliche Fruchtbarkeit besitzt, die zurückzuführen ist auf den Ersatz eines Genes, das für ein Genprodukt codiert, von dem die Microsporogenese abhängig ist, durch ein Gen, welches für das gleiche Genprodukt codiert, das jedoch in einer Expressionssequenz mit einem induzierbaren Promotor, der auf externe Kontrolle reagiert, verbunden ist, mit den folgenden Stufen:
a) Pflanzen von Samen der Pflanze, um wachsende, männlich sterile Pflanzen zu erhalten;
b) Induktion der Konversion der wachsenden Pflanze zu einer männlich fruchtbaren Form, dadurch daß man die Pflanze unter Bedingungen wachsen läßt, unter welchen der genannte Promotor induziert wird, so daß das Genprodukt, von welchem die Mikrosporogenese abhängt, produziert wird;
c) offene Befruchtung isoliert wachsender Pflanzen, um Samen zu produzieren; und
d) Ernten des Samens.

3. Verfahren zur Herstellung von Hybridsamen mit den folgenden Stufen:
a) Erhalt von Samen einer weiblichen Elternlinie, die männlich steril ist, durch ein Verfahren nach Anspruch 2;
b) Pflanzen eines ersten Samens von einer ausgewählten männlich fruchtbaren männlichen Elternlinie und eines zweiten Samens, der wie in a) beschrieben erhalten wurde, in einer Stellung, welche Kreuz-Befruchtung möglich macht;
c) Wachsenlassen des Samens zu reifen Pflanzen unter Bedingungen, unter denen die vom genannten zweiten Samen abgeleitete männliche Sterilität der Pflanze erhalten bleibt;
d) Kreuz-Befruchten der männlich sterilen weiblichen Pflanze mit Pollen der männlich fruchtbaren männlichen Pflanze; und
e) Ernten des Hybridsamens von der männlich sterilen weiblichen Pflanze.

## Revendications

1. Méthode pour fournir une fertilité mâle héréditaire, extérieurement contrôlable à une plante, comprenant les étapes consistant à :
a) sélectionner un gène qui code pour un produit génique dont dépend la microsporogenèse dans la plante ;
b) cloner le gène sélectionné ;
c) lier le gène cloné dans une séquence d'expression à un promoteur inductible répondant à un
contrôle externe ;
d) rendre inopérant le gène qui code pour le produit génique dudit gène cloné dans le génome nucléaire natif de la plante ; et
e) insérer ladite séquence d'expression dans le génome nucléaire de la plante.

2. Méthode de reproduction d'une plante ayant une fertilité mâle héréditaire, extérieurement contrôlable, résultant du remplacement d'un gène qui code pour un produit génique dont dépend la microsporogenèse par un gène qui code pour le même produit génique, mais qui est lié dans une séquence d'expression à un promoteur inductible répondant à un contrôle externe, comprenant les étapes consistant à ;
a) planter des graines de la plante pour fournir des plantes mâles-stériles en croissance ;
b) induire la conversion des plantes en croissance en une forme mâle fertile en faisant croître les plantes dans des conditions qui induisent ledit promoteur, produisant de cette manière le produit génique dont dépend la microsporogenèse ;
c) provoquer la pollinisation des plantes en croissance en les isolant pour produire des graines ; et
d) récolter les graines.

3. Méthode de production de graines hybrides comprenant les étapes consistant à :
a) obtenir des graines d'une lignée parentale femelle possédant une stérilité mâle par une méthode telle que revendiquée à la revendication 2;
b) planter en juxtaposition pour pollinisation croisée une première graine d'une lignée parentale mâle mâle fertile sélectionnée choisie et une seconde graine obtenue comme en (a) ;
c) obtenir le développement de la graine en des plantes matures dans des conditions qui conservent la stérilité mâle de la plante issue de ladite seconde graine ;
d) produire la pollinisation croisée de la plante femelle mâle stérile avec le pollen de la plante mâle mâle-fertile ; et
e) récolter des graines hybrides à partir de la plante femelle mâle-stérile.
